# EUROPÄISCHE PATENTSCHRIFT

(11) **EP 1 411 885 B1**
(45) Veröffentlichungstag und Bekanntmachung des Hinweises auf die Patenterteilung: **16.05.2007**
(21) Anmeldenummer: 02794522.9
(22) Anmeldetag: 27.07.2002
(51) Int. Cl.: A61Q 5/10, A61K 8/36

(54) **Selektive Farbveränderung keratinischer Fasern**
Selective colour change of keratinic fibres
Coloration selective des fibres kératiniques

(30) Priorität: 03.08.2001 DE 10138094
(43) Veröffentlichungstag der Anmeldung: 28.04.2004
(73) Patentinhaber: Henkel Kommanditgesellschaft auf Aktien, 40589 Düsseldorf (DE)
(72) Erfinder: HÖFFKES, Horst, 40595 Düsseldorf (DE); BERNECKER, Ullrich, 52393 Hürtgenwald (DE); HORSTMANN, Barbara, 40670 Meerbusch (DE)
(86) Internationale Anmeldenummer: PCT/EP2002/008388
(87) Internationale Veröffentlichungsnummer: WO 2003/013450

(56) Entgegenhaltungen:
- EP-A- 1 123 693
- DE-A- 4 022 847
- DE-A- 19 754 053
- GB-A- 1 278 716
- US-A- 4 357 141
- US-A- 4 698 065
- US-A- 5 376 146

## Beschreibung

Gegenstand der vorliegenden Anmeldung ist ein verfahren zur selektiven Farbveranderung keratinischer Fasern und entsprechende Mittel.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Zur Vermeidung einer Schädigung der Fasern durch wiederholte Anwendung der farbveränderenden Mittel, ist es bei vielen Applikationen wünschenswert, selektiv nur die frisch nachgewachsenen Haaransätze zu behandeln. Dieses Verfahren ist unter der Bezeichnung Ansatzbehandlung bereits seit längerem bekannt.

Die Methoden des Standes der Technik zur Ansatzbehandlung weisen aber häufig den Nachteil auf, dass die herkömmlichen Anwendungszubereitungen während der Anwendung dünnflüssiger werden und auf den Fasern verlaufen. Dies hat häufig zur Folge, dass auch bereits behandelte Teile der Fasern erneut mit diesen Zubereitungen in Kontakt kommen und dadurch geschädigt werden könnten. Anwendungszubereitungen mit höheren Anfangsviskositäten hingegen weisen den Nachteil auf, dass sie beispielsweise beim Auftragen auf die Fasern nur schlecht zu handhaben sind.

Es bestand daher die Aufgabe, Mittel zur Farbveränderung zu entwickeln, die diese Nachteile nicht aufweisen.

Es wurde nun überraschenderweise gefunden, dass diese Nachteile durch den Einsatz spezieller Anwendungszubereitungen, die in Abhängigkeit der Zeit eine Viskositätserhöhung beziehungsweise eine nur geringe Viskositäterniedrigung aufweisen, überwunden werden können.

Ein erster Gegenstand der vorliegenden Erfindung ist daher ein Mittel zur Farbveränderung keratinischer Fasern, das unmittelbar vor der Anwendung durch Mischen einer ersten alkalisch eingestellten Komponente (A) mit einer zweiten sauer eingestellten Komponente (B), enthaltend mindestens ein Oxidationsmittel, erhalten wird, dadurch gekennzeichnet, dass die zweite Komponente (B) mindestens eine verzweigte C₆- bis C₃₀-Fettsäure enthält.

Unter keratinischen Fasern werden erfindungsgemäß Pelze, Wolle, Federn und insbesondere menschliche Haare verstanden.

Im Rahmen der Farbveränderungen sind selektive Farbveränderungen erfindungsgemäß besonders bevorzugt. Unter einer selektiven Farbveränderung werden erfindungsgemäß alle Prozesse verstanden, bei denen einzelne Fasersträhnen in voller Länge oder ausgewählte Abschnitte einzelner beziehungsweise aller Fasern behandelt werden sollen. Der häufigste Fall einer derartigen Anwendung ist die sogenannte Ansatzbehandlung der frisch nachgewachsenen Fasern. Eine weiterer Anwendungsbereich für die erfindungsgemäßen Mittel ist die sogenannten Strähnchenapplikation, bei der einzelne Strähnen gezielt eine andere Färbung als die restlichen Fasern erhalten sollen. Prinzipiell ist es aber auch denkbar, mit Hilfe des erfindungsgemäßen Mittels Mittelstücke der Fasern selektiv zu behandeln und auf diese Weise gezielt Muster in die Fasern einzuarbeiten.

Mit Hilfe der erfindungsgemäßen Mittel ist es möglich, eine gezielte Ansatzbehandlung durchzuführen. Durch das besondere Viskositätsverhalten der erfindungsgemäßen Mittel werden die Vorteile einer niedrigviskosen Anwendungszubereitung während des Auftragens (leicht Handhabbarkeit) mit denen einer höherviskosen Zubereitung während der Einwirkzeit auf den Fasern (geringes Verlaufen, gezielte Anwendungsbereiche) verbunden.

### Säuren

Die erfindungsgemäßen Mittel enthalten mindestens eine verzweigte C₆- bis C₃₀-Fettsäure. Erfindungsgemäß sind gesättigte verzweigte C₆- bis C₃₀-Fettsäuren bevorzugt.

Erfindungsgemäß besonders bevorzugte verzweigte C₆- bis C₃₀-Fettsäuren sind insbesondere 2-Ethylhexansäure, Isotridecansäure, Isopalmitinsäure, Isostearinsäure, 2-Hexyldecansäure und 2-Octyldodecansäure.

Ganz besonders bevorzugte sind die Isostearinsäuren, die beispielsweise unter den Bezeichnungen Emersol^{®} 871 und Emersol^{®} 875 vertrieben werden, und die Isopalmitinsäuren, wie beispielsweise das Handelsprodukt Edenor^{®} IP 95. Die Isostearinsäure ist eine besonders bevorzugte C₆- bis C₃₀-Fettsäure.

In einer zweiten Ausführungsform enthalten die in erfindungsgemäßen Verfahren verwendeten Mittel mindestens eine verzweigte oder unverzweigte C₈- bis C₃₀-Dicarbonsäure. Obwohl prinzipiell alle C₈- bis C₃₀-Dicarbonsäuren erfindungsgemäß geeignet sind, haben sich
- die C₈- bis C₃₀-Dicarbonsäuren der Formel (I) 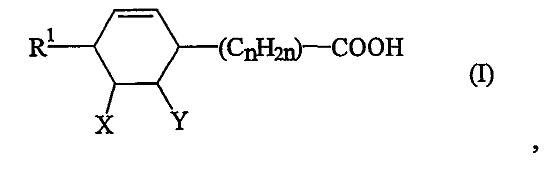 in der R¹ steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest.
- die Dicarbonsäuren der allgemeinen Formel (I), die zusätzlich noch 1 bis 3 Methyl- oder Ethylsubstituenten am Cyclohexenring tragen und
- die Dicarbonsäuren, die aus den Carbonsäuren gemäß Formel (I) formal durch Anlagerung eines Moleküls Wasser an die Doppelbindung im Cyclohexenring entstehen, als besonders geeignet erwiesen,
mit der Maßgabe, dass die Substituenten derart gewählt sind, dass die Dicarbonsäure maximal 30 C-Atome enthält.

Die Carbonsäuren der Formel (I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Dabei bietet sich vor allen die aus natürlichen Fetten und Ölen zugängliche Linolsäure an. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Es sind solche Dicarbonsäuren der Formel (I) bevorzugt, bei denen R¹ steht für eine lineare oder methylverzweigte, gesättigte Alkylgruppe mit 4 bis 8 Kohlenstoffatomen und n für eine Zahl von 6 bis 10. Ganz besonders bevorzugt sind solche Dicarbonsäuren der Formel (I), bei denen R¹ steht für eine Hexylgruppe, n steht für 7, einer der Substituenten X oder Y steht für eine Carboxygruppe und der andere der Substituenten X oder Y steht für Wasserstoff.

In erfindungsgemäßen Verfahren einsetzbar neben den, bevorzugten, Dicarbonsäuren gemäß Formel (I) sind auch solche Dicarbonsäuren, die sich von den Verbindungen gemäß Formel (I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 8-(2'-Carboxy-4'-hexyl-cyclohex-5'-en)-1-octansäure und 8-(2'-Carboxy-4'-hexyl-cyclohex-5'-en)-1-octansäure. Solche Verbindungen sind kommerziell unter den Bezeichnungen Westvaco Diacid^{®}1550 und Westvaco Diacid^{®} 1595 (Hersteller: Westvaco) erhältlich.

### Oxidationsmittel

Das erfindungsgemäße Mittel eignet sich insbesondere zur selektiven oxidativen Farbveränderung in Form einer oxidativen Färbung oder einer Blondierung. In beiden Fällen enthält die Komponente (B) ein Oxidationsmittel. Als Oxidationsmittel kommen bevorzugterweise Persulfate, Chlorite und insbesondere Wasserstoffperoxid oder dessen Anlagerungsprodukte an Harnstoff, Melamin sowie Natriumborat in Frage. Als Komponente (B) haben sich wäßrige Wasserstoffperoxidlösungen erfindungsgemäß als besonders geeignet erwiesen. Weiterhin ist es möglich, die Oxidation mit Hilfe von Enzymen zu beschleunigen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Substraten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Unabhängig davon, welche der oben genannten Varianten im Rahmen der erfindungsgemäßen Farbveränderung gewählt wird, kann die Ausbildung der Färbung dadurch unterstützt und gesteigert werden, dass dem Mittel bestimmte Metallionen zugesetzt werden. Solche Metallionen sind beispielsweise Zn²⁺, Cu⁺, Cu²⁺, Fe²⁺, Fe³⁺, Mn²⁺,Ru³⁺, Mn⁴⁺, Li⁺, Mg²⁺, Ca²⁺ und Al³⁺. Besonders geeignet sind dabei Zn²⁺, Cu²⁺, Ru³⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

### Nichtionische Tenside

In einer weiteren bevorzugten Ausführungsform kann die Wirkung der erfindungsgemäßen Mittel durch nichtionische Tenside gesteigert werden. Nichtionische Tenside enthalten als hydrophile Gruppe z. B. eine Polyolgruppe, eine Polyalkylenglykolethergruppe oder eine Kombination aus Polyol- und Polyglykolethergruppe.

Bevorzugt können die erfindungsgemäßen Zusammensetzungen mindestens ein nichtionisches Tensid mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionische Tenside mit einem HLB-Wert von 10-17 können erfindungsgemäß besonders bevorzugt sein.

Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Ethoxylierung- und Propoxylierungsprodukte von Polyhydroxyfettsäuren,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (NT1)

   R¹CO-(OCH₂CHR²)_{w}OR³ Formel (NT1),

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 197 38 866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bevorzugte nichtionische Tenside sind ausgewählt aus
- Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diestern von Anlagerungsprodukten von 1 bis 30 Mol Ethylenoxid an Glycerin,
- Ethoxylierung- und Propoxylierungsprodukte von Polyhydroxyfettsäuren,
- Anlagerungsprodukten von 5 bis 60 Mol Ethylenoxid an Rizinusöl und gehärtetes Rizinusöl,
- Polyolfettsäureestern und
- Sorbitanfettsäureestern.

Besonders bevorzugte nichtionische Tenside sind Anlagerungsprodukte von 2 bis 50 Mol Ethylenoxid und/oder 0 bis 5 Mol Propylenoxid an lineare und verzweigte Fettalkohole mit 8 bis 30 C-Atomen.

Als ganz besonders bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Derartige Emulgatoren sind beispielsweise unter den Bezeichnungen Eumulgin® B 1 und Eumulgin® B2 (Cognis) im Handel erhältlich.

Ebenfalls als ganz besonders bevorzugte nichtionische Tenside haben sich Ethoxylierung- und Propoxylierungsprodukte von Polyhydoxyfettsäuren erwiesen. Derartige Emulgatoren sind beispielsweise unter der Bezeichnung Arlacel^{®} P135 (INCI-Bezeichnung: PEG-30 Dipolyhydroxystearate) im Handel erhältlich.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die nichtionischen Tenside werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

Für die Erzielung der erfindungsgemäßen Wirkung spielt es keine wesentliche Rolle, ob die nichtionischen Emulgatoren in der Komponente (A) oder der Komponente (B) enthalten sind. Es kann aber bevorzugt sein, wenn die Komponente (B) mindestens einen nichtionischen Emulgator enthält. Weiterhin kann es bevorzugt sein, wenn sowohl die Komponente (A) als auch die Komponente (B) mindestens einen nichtionischen Emulgator enthält.

### Verdickungsmittel

In einer weiteren bevorzugten Ausführungsform der vorliegenden Erfindung enthält die Komponente (B) mindestens ein Verdickungsmittel. Bezüglich dieser Verdickungsmittel bestehen keine prinzipiellen Einschränkungen. Obwohl prinzipiell sowohl organische als auch rein anorganische Verdickungsmittel zum Einsatz kommen können, sind die organischen Verdickungsmittel erfindungsgemäß bevorzugt.

Gemäß einer ersten, bevorzugten, Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidon, Vinylether und Vinylester.

Bevorzugte anionische Copolymere sind beispielsweise Copolymere aus Acrylsäure, Methacrylsäure oder deren C₁- bis C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁- bis C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Compounds, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnungg Stabileze^{®} QM im Handel erhältlich.

Gemäß einer zweiten Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (V1), 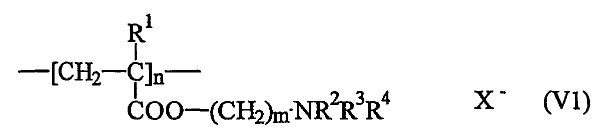 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (V1) aufgeführten Monomereinheiten sowie nichtionogenen Monomereinheiten, sind besonders bevorzugte kationische polymere Verdickungsmittel. Im Rahmen dieser Polymeren sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Monnitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6) im Handel erhältlich.

Copolymere mit Monomereinheiten gemäß Formel (V1) enthalten als nichtionogene Monomereinheiten bevorzugt Acrylamid, Methacrylamid, Acrylsäure-C₁₋₄-Alkylester und Methacrylsäure-C₁₋₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid- Methacryloyloxyethyltrimethylammoniumchlorid-Copolymer. Solche Copolymere, bei denen die Monomeren in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

In einer dritten bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben. Erfindungsgemäß bevorzugte modifizierte Guargums enthalten C₁- bis C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guar Gums sind unter den Handelsbezeichnungen Jaguar^{®} HP8, Jaguar^{®} HP60, Jaguar^{®} HP 120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt. Es wird daher explizit auf das Werk von Robert L. Davidson mit dem Titel "Handbook of Water soluble gums and resins", erschienen bei Mc Graw Hill Book Company (1980) verwiesen.

Gemäß dieser Ausführungsform bevorzugt sind weiterhin Biosaccharidgums mikorbiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine.

Besonders bevorzugte natürliche Polymere sind die Xanthangums.

Aber auch nichtionische, vollsynthetische Polymere, wie beispielsweise Polyvinylalkohol oder Polyvinylpyrrolidon, sind als erfindungsgemäße Verdickungsmittel einsetzbar. Bevorzugte nichtionische, vollsynthetische Polymere werden beispielsweise von der Firma BASF unter dem Handelsnamen Luviskol^{®} vertrieben.

In einer weiteren Ausführungsform der vorliegenden Erfindung werden als Verdickungsmittel Cellulose und/oder Cellulosederivate, wie beispielsweise Methylcellulose, Carboxyalkylcellulosen und Hydroxyalkylcellulosen eingesetzt.

Bevorzugte Hydroxyalkylcellulosen sind insbesondere die Hydroxyethylcellulosen, die unter den Bezeichnungen Cellosize^{®} der Firma Amerchol, Natrosol^{®} der Firma Hercules oder Culminal^{®} und Benecel^{®} der Firma Aqualon vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

### Oxidative Haarfärbung

In einer zweiten bevorzugten Ausführungsform der vorliegenden Erfindung wir das erfindungsgemäße Mittel im Rahmen einer oxidativen Haarfärbung verwendet. Dabei wird unmittelbar vor der Anwendung auf dem Haar das Färbemittel (Komponente (A)) mit einer Oxidationsmittelzubereitung (Komponente (B)) vermischt.

Oxidative Haarfärbemittel enthalten in der Komponente (A) erfindungsgemäß mindestens ein Farbstoffvorprodukt ausgewählt aus
- den Oxidationsfarbstoffvorprodukten vom Entwickler- und Kupplertyp und/oder
- den Farbstoffvorprodukte vom Indolin- und Indoltyp
enthalten.

Die Entwicklerkomponenten bilden unter dem Einfluß von Oxidationsmitteln, gegebenenfalls mit Hilfe spezieller Enzyme oder Metallionen als Katalysator, untereinander oder unter Kupplung mit einer oder mehreren Kupplerkomponenten die eigentlichen Farbstoffe aus.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2',5'-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4'-aminophenyl)-diazacycloheptan, 1,3-Bis(N(2'-hydroxyethyl)-N(4'-aminophenylamino))-2-propanol, 4-Amino-2-(2'-hydroxyethoxy)-phenol, 1,10-Bis-(2',5'-diaminophenyl)-1,4,7,10-tetraoxadecan sowie 4,5-Diaminopyrazol-Derivate nach EP 0 740 931 B1 bzw. WO 94/08970 A1 wie z.B. 4,5-Diamino-1-(2'-hydroxyethyl)-pyrazol.

Erfindungsgemäß besonders bevorzugt sind die Entwicklerkomponenten p-Phenylendiamin, p-Toluylendiamin, p-Aminophenol, 1-(2'-Hydroxyethyl)-2,5-diaminobenzol, N,N-Bis-(2'-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methykesorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol und
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Weitere in oxidativen Färbemitteln enthaltene Farbstoffvorprodukte können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Ferner können die oxidativen Färbemittel zur weiteren Nuancierung verschiedene direktziehende Farbstoffe enthalten.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die Färbezubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Das anwendungsbereite Haarfärbemittel ist üblicherweise alkalisch, d. h. auf pH-Werte im Bereich von etwa 7 bis 11, eingestellt. Zu diesem Zweck enthalten die Färbemittel (Komponente (A)) Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propan-1-ol, 2-Amino-2-methyl-1,3-propandiol, 2-Amino-2-ethyl-1,3-propandiol, 2-Aminc-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

### Blondiermittel

In einer ersten bevorzugten Ausführungsform wird das erfindungsgemäße Mittel im Rahmen einer Blondierung eingesetzt.

Im Rahmen eines derartigen Blondierverfahrens wird die Anwendungszubereitung durch Mischung eines sogenannten Blondiermittels (Komponente (A)) mit einer Wasserstoffperoxidzubereitung (Komponente (B)) erhalten. Die Blondiermittel können zusätzlich feste Anlagerungsverbindung von Wasserstoffperoxid an anorganische oder organische Verbindungen, wie beispielsweise Natriumperborat, Natriumpercarbonat, Natriumpercarbamid, Polyvinylpyrrolidon, Harnstoffperoxid und Melaminperoxid enthalten.

Die Blondierwirkung kann durch sogenannte "Booster" gesteigert werden. Dies sind in der Regel feste Peroxoverbindung, die keine Anlagerungsprodukte von Wasserstoffperoxid an andere Komponenten darstellen. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Peroxoverbindungen sind in den erfindungsgemäßen Blondiermitteln bevorzugt in Mengen von 5-30 Gew.-%, insbesondere in Mengen von 8-20 Gew.-%, enthalten.

Als weitere wichtige Komponente enthalten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Blondiermittel enthalten Alkalisierungsmittel bevorzugt in Mengen von 5-25 Gew.-%, insbesondere 10-20 Gew.-%.

Für die Anwendung der Mittel auf dem Haar werden die Blondiermittel unmittelbar vor dem Auftragen mit einer Wasserstoffperoxidlösung vermischt. Die Konzentration dieser Wasserstoffperoxidlösung wird einerseits von den gesetzlichen Vorgaben und andererseits von dem gewünschten Effekt bestimmt; in der Regel werden 6- bis 12-prozentige sauer eingestellte Lösungen in Wasser verwendet. Die Mengenverhältnisse von Blondiermittel und Wasserstoffperoxidlösung liegen dabei üblicherweise im Bereich 1:1 bis 1:2, wobei ein Überschuß an Wasserstoffperoxidlösung insbesondere dann gewählt wird, wenn keine zu ausgeprägte Blondierwirkung erwünscht ist.

### Tenside

In einer weiteren bevorzugten Ausführungsform enthalten die erfindungsgemäßen Mittel mindestens ein Tensid. Unter dem Begriff Tenside werden erfindungsgemäß oberflächenaktive Substanzen verstanden, die anionischer, kationischer zwitterionischer oder amphoterer Natur sein können.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)x-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 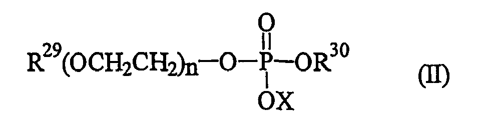 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄-Kohlenwasserstoffrest, steht,
- sulfatierte Fettsäurealkylenglykolester der Formel (III)

   R³⁵CO(AlkO)ₙSO₃M (III)

   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV) wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind. 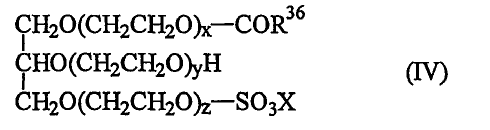 in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (IV) eingesetzt, in der R³⁶CO für einen linearen Acylrest mit 8 bis 18 Kohlenstoffatomen steht

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Die Tenside werden in Mengen von 0,1 - 45 Gew.-%, bevorzugt 1 - 30 Gew.-% und ganz besonders bevorzugt von 1-15 Gew.-%, bezogen auf das gesamte Mittel, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Unter den genannten Emulgatoren-Typen können die Emulgatoren, welche kein Ethylenoxid und/oder Propylenoxid im Molekül enthalten ganz besonders bevorzugt sein.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

### Anwendungsbedingungen

Ein weiterer Gegenstand der vorliegenden Erfindung ist ein Verfahren zur selektiven Farbveränderung keratinischer Fasern, bei dem
- in einem ersten Schritt die Komponenten (A) und (B) der erfindungsgemäßen Mittel zu einer Anwendungszubereitung (C) vermischt werden,
- in einem zweiten Schritt die resultierende Anwendungszubereitung (C) selektiv auf einzelne Bereiche der Fasern aufgetragen wird,
- gegebenenfalls nach einer Einwirkungszeit in einem dritten Schritt die gesamten Fasern einer farbverändernden Behandlung unterzogen werden und
- nach einer Einwirkungszeit die Fasern gespült werden,
dadurch gekennzeichnet, dass die zweite Komponente (B) mindestens eine verzweigte C₆ - bis C₃₀ - Fettsäure und/oder mindestens eine verzweigte oder unverzweigte C₈ - bis C₃₀ - Dicarbonsäure enthält.

Die Anwendungstemperaturen können im Rahmen des vorliegenden Verfahrens in einem Bereich zwischen 15 und 40 °C, bevorzugt bei der Temperatur der Kopfhaut, liegen. Die Einwirkungszeit nach der zweiten Stufe des erfindungsgemäßen Verfahrens beträgt bevorzugt zwischen 5 und 45, insbesondere 15 bis 30, Minuten.

In einer ersten Ausführungsform des vorliegenden Verfahrens wird die gesamte Anwendungszubereitung im unmittelbaren Anschluss an die erste Einwirkungszeit mit Wasser und gegebenenfalls einem geeigneten Shampoo gründlich ausgewaschen (Verfahren ohne den dritten Schritt). Dies ist häufig insbesondere bei Blondierverfahren der Fall, bei denen die Haarspitzen noch von der letzten Behandlung eine unveränderte Färbung aufweisen.

In einer anderen Ausführungsform des erfindungsgemäßen Verfahrens kann es aber auch wünschenswert sein, abschließend die gesamte Faserlängen einer Farbveränderung zu unterziehen. Dieses Verfahren spielt insbesondere bei der Ansatzbehandlung, bei der die frisch nachgewachsenen Ansätze einer intensiveren Behandlung als die bereits behandelten Faserlängen bedürfen, eine wesentliche Rolle. Zu diesem Zweck kann nach Ende der Einwirkungszeit entweder die Anwendungszubereitung beispielsweise mit einem Kamm auf der gesamten Faserlänge verteilt werden oder es kann noch zusätzliche Anwendungszubereitung auf die im Rahmen des vorliegenden Verfahrens noch unbehandelten Faserabschnitte aufgetragen werden. Dabei spielt es für den Erfolg des erfindungsgemäßen Verfahrens keine Rolle, ob diese Anwendungszubereitung identisch oder unterschiedlich zu der der selektiven Vorbehandlung formuliert ist. Auch im Rahmen dieser Ausführungsform werden die Fasern abschließend mit Wasser und gegebenenfalls einem Shampoo gründlich gespült.

Das Nachwaschen mit einem Shampoo entfällt, wenn ein stark tensidhaltiger Träger, z. B. ein Färbeshampoo, verwendet wurde.

In einer bevorzugten Ausführungsform der vorliegenden Erfindung ist die Zubereitung (A) gelförmig. Unter gelförmigen Zubereitungen werden erfindungsgemäß formstabile Massen bezeichnet, die unter Druck deformierbar sind, das heißt eine Fließgrenze aufweisen. Dieser Effekt ist insbesondere bei Zubereitungen zu beobachten, die eine dreidimensionale Netzwerkstruktur aufweisen.

In einer weiteren Ausführungsform der vorliegenden Erfindung wird die Zubereitung (B) in Form einer O/W-Emulsion oder einer W/O-Emulsion formuliert.

Die folgenden Beispiele sollen den Anmeldungsgegenstand näher erläutern:

### Beispiele

Die folgenden Mengenangaben sind, soweit nichts anderes vermerkt ist, in Gewichtsprozent

### Beispiel I

### 1. Oxidationsmittelzubereitungen

Es wurden die folgenden Oxidationsmittelzubereitungen hergestellt:

| Rohstoff | Vergleichsrezeptur V1 | Beispiel E1.1 | Beispiel E1.2 |
|---|---|---|---|
| Texapon^{®} N28¹ | 2,0 | 2,0 | 2,0 |
| Dipicolinsäure | 0,1 | 0,1 | 0,1 |
| Natriumpyrophosphat | 0,03 | 0,03 | 0,03 |
| Turpinal^{®} SL² | 1,5 | 1,5 | 1,5 |
| Dow Corning^{®} DB 110 A³ | 0,07 | 0,07 | 0,07 |
| Aculyn^{®} 33⁴ | 12,0 | 12,0 | 12,0 |
| Eumulgin^{®} B1⁵ | --- | 0,5 | --- |
| Isostearinsäure | --- | 1,5 | 2,0 |
| Wasserstoffperoxid (50%ig) | 12,0 | 12,0 | 12,0 |
| Ammoniak, 25%ig | ad pH 4 | ad pH 4 | ad pH 4 |
| Wasser | ad 100 | ad 100 | ad 100 |
| | | | |

| Rohstoff | Rezeptur V2 | Rezeptur E2.1 | Rezeptur E2.2 |
|---|---|---|---|
| Natronlauge (45%ig) | 0,7 | 0,7 | 0,7 |
| Dipicolinsäure | 0,1 | 0,1 | 0,1 |
| Natriumpyrophosphat | 0,03 | 0,03 | 0,03 |
| Texapon^{®} NSO F⁶ | 2,0 | 2,0 | 2,0 |
| Aculyn^{®} 33 | 5,5 | 5,5 | 5,5 |
| Turpinal^{®} SL | 1,5 | 1,5 | 1,5 |
| Eumulgin^{®} B1 | --- | 0,5 | --- |
| Isostearinsäure | --- | 1,5 | 2,0 |
| Wasserstoffperoxid (50%ig) | 10,0 | 10,0 | 10,0 |
| Wasser | ad 100 | ad 100 | Ad 100 |

| | | | |
|---|---|---|---|
| ¹ Laurylethersulfat-Natrium-Salz (INCI-Bezeichnung: Sodium Laureth Sulfate, ca. 26.5% Aktivsubstanz) (Cognis) ² 1-Hydroxyethan-1,1-diphosphonsäure (INCI-Bezeichnung: Etidronic Acid) (Cognis) ³ nichtionogene Silicon Emulsion (INCI-Bezeichnung: Dimethicone, ca. 10% Aktivsubstanz) (Dow Coming) ⁴ Säure-enthaltendes, vernetztes Acrylcopolymer (INCI-Bezeichnung: Acrylates Copolymer, ca. 28% Aktivsubstanz) (Rohm&Haas) ⁵ C₁₆₋₁₈-Fettalkohol mit ca. 12-EO-Einheiten (INCI-Bezeichnung: Ceteareth-12) (Cognis) ⁶ Laurylethersulfat-Natrium-Salz (INCI-Bezeichnung: Sodium Laureth Sulfate, 27% bis 29% Aktivsubstanzgehalt) (Cognis) | | | |

### 2. Färbecremes

Es wurden die folgenden Färbecremes hergestellt:

| Rohstoff | Rezeptur 2.1 |
|---|---|
| Hydrenol^{®} D⁷ | 5,5 |
| Kokoslorol⁸ | 2,0 |
| Eutanol^{®} G⁹ | 1,0 |
| Eumulgin^{®} B1 | 0,5 |
| Eumulgin^{®} B2¹⁰ | 0,5 |
| Lamesoft^{®} PO 65¹¹ | 2,0 |
| Texapon^{®} K 14 S 70 C¹² | 2,8 |
| Akypo Soft^{®} 45 NV¹³ | 10,0 |
| Polymer W 37194¹⁴ | 3,8 |
| Natriumsulfit | 0,5 |
| L(+)-Ascorbinsäure, reinst | 0,4 |
| p-Toluylendiamin-sulfat | 1,5 |
| 2-Methylresorcin | 1,1 |
| Resorcin | 0,3 |
| Methylgelb¹⁵ | 0,05 |
| 2,4,5,6-Tetraaminopyrimidin -sulfat | 1,7 |
| m-Aminophenol | 0,07 |
| 4-Amino-3-methylphenol | 0,1 |
| 2,7-Dihydroxynaphthalin | 0,5 |
| Parfüm | 0,3 |
| Turpinal^{®} SL | 0,2 |
| Ammoniak (25%ig) | 8,0 |
| Wasser | ad 100 |

| | |
|---|---|
| ⁷ C₁₆₋₁₈-Fettalkohol (INCI-Bezeichnung: Cetearyl Alcohol) (Cognis) ⁸ C12-18-Fettalkohol (INCI-Bezeichnung: Coconut Alcohol) (Cognis) ⁹ 2-Octyldodecylalkohol (INCI-Bezeichnung: Octyldodecanol) (Cognis) ¹⁰ Cetylstearylalkohol mit ca. 20-EO-Einheiten (INCI-Bezeichnung: Ceteareth-20) (Cognis) ¹¹ Alkylpolyglucosid-Ölsäuremonoglycerid-Gemisch (INCI-Bezeichnung: Coco-Glucoside, Glyceryl Oleate, Aqua (Water); ca. 32% bis 35% Wassergehalt) (Cognis) ¹² Laurylmyristylethersulfat-Natrium-Salz (ca. 68% bis 73% Aktivsubstanzgehalt'; INCI-Bezeichnung: Sodium Myreth Sulfate) (Cognis) ¹³ Laurylalkohol-4.5-EO-Essigsäure-Natrium-Salz (mind. 21% Aktivsubstanzgehalt; INCI-Bezeichnung: Sodium Laureth-6 Carboxylate) (Chem-Y) ¹⁴ Acrylsäure-Natrium-Salz-Acrylamidopropyltrimethylammoniumchlorid-Copolymer; konserviert mit Phenonip (19% bis 21% Aktivsubstanzgehalt; INCI-Bezeichnung: Acrylamidopropyltrimonium Chloride/Acrylates Copolymer) (Stockhausen) ¹⁵ 2-(4'-Methyl-2'-nitrophenyl)aminoethanol | |

| Rohstoff | Rezeptur 2.2: | Rezeptur 2.3: |
|---|---|---|
| Edenor^{®} PK 1805¹⁶ | 6,8 | 6,8 |
| Texapon^{®} NSO UP¹⁷ | 3,5 | 3,5 |
| 1,2-Propylenglykol | 6,8 | 7,2 |
| Kokoslorol | 8,0 | 8,0 |
| Dehydol^{®} LS 2¹⁸ | 10,1 | 10,1 |
| Gluadin^{®} W 40¹⁹ | 3,0 | 3,0 |
| Isopropanol | 14,0 | 14,0 |
| L-Arginin | 1,0 | 1,0 |
| L(+)-Ascorbinsäure, reinst | 0,2 | 0,2 |
| Natriumsulfit | 0,2 | 0,2 |
| Turpinal^{®} SL | 0,2 | 0,2 |
| p-Toluylendiamin-sulfat | | 0,8 |
| Resorcin | 1,9 | 0,1 |
| Methylgelb | | 0,1 |
| 3-Amino-2-methylamino-6-methoxypyridin-hydrochlorid | 0,1 | -- |
| 4-Amino-3-methylphenol | -- | 0,2 |
| 5-Amino-2-methylphenol | -- | 0,1 |
| Monoethanolamin | 6,5 | 4,5 |
| p-Toluylendiamin | 2,1 | -- |
| 1,3-Bis-(2',4'-diaminophenoxy)-propan | 0,1 | -- |
| Parfüm | 0,6 | 0,6 |
| Wasser | ad 100 | ad 100 |

| | | |
|---|---|---|
| ¹⁶ Ölsäure (INCI-Bezeichnung: Oleic Acid) (Cognis) ¹⁷ Natriumlaurylethersulfat (26,5% bis 27,5% Aktivsubstanzgehalt; INCI-Bezeichnung: Sodium Laureth Sulfate) (Cognis) ¹⁸ C₁₂₋₁₄-Fettalkohol mit ca. 2-EO-Einheiten (INCI-Bezeichnung: Laureth-2) (Cognis) ¹⁹ Weizenproteinhydrolysat (ca. 40% Aktivsubstanz; INCI-Bezeichnung: Aqua (Water), Hydrolyzed Wheat Protein, Sodium Benzoate, Phenoxyethanol, Methylparaben, Propylparaben) (Cognis) | | |

### 3. Ausfärbungen

### Versuchsreihe A

Jeweils 50g der Färbecreme 2.1 wurden mit jeweils 50g der Oxidationsmittelzubereitungen V1, E1.1 und E1.2 vermischt.

### Beurteilung der rheologischen Eigenschaften

Unmittelbar nach dem Vermischen wiesen die erfindungsgemäßen Beispiele (Oxidationsmittelzubereitungen E1.1 und E1.2) eine niedrigere Viskosität auf als die Vergleichsrezeptur. Bei Überprüfung auf einer senkrecht gestellten Glasscheibe war dies durch eine höhere Fließgeschwindigkeit der erfindungsgemäßen Formulierungen zu erkennen. Bei den Versuchen zur Überprüfung der Fließgeschwindigkeiten wurde jeweils 1g der Zubereitungen auf einer markierten Startlinie in einem Abstand von 4cm auf einer Glasscheibe aufgetragen. Unmittelbar nach dem Aufbringen der Zubereitungen wurde die Scheibe senkrecht gestellt und das Fließverhalten beobachtet.

Nach einer Standzeit von 20 Minuten bei Raumtemperatur wiesen die erfindungsgemäßen Formulierungen nunmehr eine höhere Viskosität auf als die Vergleichrezeptur. An der senkrecht gestellten Glasscheibe wiesen die erfindungsgemäßen Rezepturen eine deutlich niedrigere Fließgeschwindigkeit als die Vergleichsrezeptur auf.

### Versuchsreihe B

Jeweils 50g der Färbecreme 2.2 wurden mit jeweils 40g der Oxidationsmittelzubereitungen V2, E2.1 und E2.2 vermischt.

### Versuchsreihe C

Jeweils 50g der Färbecreme 2.3 wurden mit jeweils 40g der Oxidationsmittelzubereitungen V2, E2.1 und E2.2 vermischt.

Die erfindungsgemäßen Anwendungszubereitungen der Versuchsreihen B und C wiesen einen optimierten Viskositätsverlauf in Abhängigkeit von der Zeit auf.

### Beispiel II

Es wurden die folgenden Entwickleremulsionen hergestellt:

Wässrige Phase:

| Rohstoff | Menge |
|---|---|
| Wasserstoffperoxidlösung (50%ig) | 30,0g |
| Turpinal^{®} SL | 2,4g |
| MgSO₄ · 7 H₂O | 0,8g |
| Dipicolinsäure | 0,2g |
| Aerosil^{®} 200 V²⁰ | 1,2g |
| Wasser | ad 240g |

| | |
|---|---|
| ²⁰ verdichtete Kieselsäure (INCI-Bezeichnung: Silica) (Degussa) | |

Fettphase:

| Rohstoff | Rezeptur 1 | Rezeptur 2 |
|---|---|---|
| Eumulgin^{®} B1 | 6,5g | -- |
| Eumulgin^{®} B2 | 6,5g | -- |
| Arlacel^{®} P 135²¹ | -- | 13,0 |
| Cetiol^{®} S²² | 11,2g | 11,2g |
| Cetiol^{®} B²³ | 3,7g | 3,7g |
| Myritol^{®} 318²⁴ | 3,7 g | 3,7 g |
| Isostearinsäure | 11,2g | 11,2g |
| Ölsäure | 3,7g | 3,7g |
| Myristinsäure | 3,7g | 3,7g |
| Paraffinöl, dünnflüssig | ad 80g | ad 80g |

| | | |
|---|---|---|
| ²¹ Polyethylenglykol-di-polyhydroxystearat (INCI-Bezeichnung: PEG-30 Dipolyhydroxystearate) (Unichema) ²² 1,3-Bis(2-ethylhexyl)cyclohexan (INCI-Bezeichnung: Diethylhexylcyclohexane) (Cognis) ²³ Adipinsäuredibutylester (INCI-Bezeichnung: Dibutyl Adipate) (Cognis) ²⁴ Fettsäuretriglycerid (INCI-Bezeichnung: Caprylic/Capric Triglyceride) (Cognis) | | |

Die Fettphasen wurden jeweils auf ca. 70 bis 80°C, die wässrige Phase auf ca. 60°C erhitzt. Unter Rühren (200 UPM) wurden jeweils 120g der wässrigen Phase zu 80g der jeweiligen Fettphase gegeben. Anschließend wurden die gebildeten Emulsionen unter Rühren (100 UPM) auf 35°C abgekühlt.
Die Mischung mit der Fettphase der Rezeptur 1 ergab eine niedrigviskose O/W-Emulsion, die Mischung mit der Fettphase der Rezeptur 2 ergab eine ebenfalls niedrigviskose W/O-Emulsion.

Beide Entwickleremulsionen wurden im Verhältnis 1:1 mit der folgenden Färbecremerezeptur vermischt:

| Rohstoff | Menge |
|---|---|
| Stenol^{®} 1618²⁵ | 9,0 |
| Kokosfettalkohol | 3,0 |
| Texapon^{®} NSO F | 7,0 |
| Dehyton^{®} K²⁶ | 5,0 |
| Prisorine^{®} 3501²⁷ | 2,0 |
| Edenor^{®} C 14 98/100²⁸ | 0,5 |
| Eumulgin^{®} B1 | 0,5 |
| Eumulgin^{®} B2 | 0,5 |
| wässrige Kalilauge (50%ig) | 1,2 |
| Natriumsulfit | 0,5 |
| Ammoniumsulfat | 0,3 |
| 5-Amino-2-methylphenol | 0,02 |
| 2,4,5,6-Tetraaminopyrimidin-sulfat | 1,92 |
| Resorcin | 0,15 |
| 2-Methylresorcin | 0,34 |
| p-Toluylendiamin-sulfat | 0,09 |
| 2,7-Dihydroxynaphthalin | 0,42 |
| 2,6-Dihydroxy-3,4-dimethylpyridin | 0,10 |
| 1,3-Bis-(2,4-diaminophenoxy)-propan-tetrahydrochlorid | 0,05 |
| Turpinal^{®} SL | 0,2 |
| Parfüm | 0,30 |
| wässrige Ammoniaklösung (25%ig) | ad pH 10 |
| Wasser | ad 100 |

| | |
|---|---|
| ²⁵ C₁₆₋₁₈-Fettalkohol (INCI-Bezeichnung: Cetearyl Alcohol) (Cognis) ²⁶ N,N-Dimethyl-N(C₈₋₁₈-kokosamidopropyl)ammoniumacetobetain (ca. 29-32% Aktivsubstanzgehalt; INCI-Bezeichnung: Aqua (Water), Cocamidopropyl Betaine) (Cognis) ²⁷ Isostearinsäure (Isomerengemisch) (INCI-Bezeichnung: Isostearic Acid) (Unichema) ²⁸ Myristinsäure (INCI-Bezeichnung: Myristic Acid) (Cognis) | |

Beim Vermischen mit der Entwickleremulsion der Rezeptur 1 entstand eine geschmeidige Creme, beim Vermischen mit der Entwickleremulsion der Rezeptur 2 entstand eine viskose Paste.
In beiden Fällen resultierten brillante, intensiv rote Ausfärbungen auf Haarsträhnen (Kerling, naturweiß).

## Patentansprüche

1. Verfahren zur selektiven Farbveränderung keratinischer Fasern, bei dem
- in einem ersten Schritt eine erste alkalisch eingestellte Komponente (A) mit einer zweiten sauer eingesteliten Komponente (B), enthaltend mindestens ein Oxidationsmittel, zu einer Anwendungszubereitung (C) vermischt werden,
- in einem zweiten Schritt die resultierende Anwendungszubereitung (C) selektiv auf einzelne Bereiche der Fasern aufgetragen wird,
- gegebenenfalls nach einer Einwirkungszeit in einem dritten Schritt die gesamten Fasern einer farbverändernden Behandlung unterzogen werden und
- nach einer Einwirkungszeit die Fasern gespült werden,
**dadurch gekennzeichnet, dass** die zweite Komponente (B) mindestens eine verzweigte C_{G}- bis C₃₀-Fettsäure und/oder mindestens eine verzweigte oder unverzweigte C₈- bis C₃₀-Dicarbonsäure enthält.

2. Verfahren nach Anspruch 1, **dadurch gekennzeichnet, dass** die verzweigte C₆- bis C₃₀-Fettsäure gesättigt ist.

3. Verfahren nach Anspruch 2, **dadurch gekennzeichnet, dass** die verzweigte C₆- bis C₃₀-Fettsäure ausgewählt ist aus 2-Ethylhexansäure, Isotridecansäure. Isopalmitinsäure, Isostearinsäure, 2-Hexyldecansäure und 2-Octyldodecansäure.

4. Verfahren nach Anspruch 3, **dadurch gekennzeichnet, dass** die verzweigte C₆- bis C₃₀-Fettsäure Isostearinsäure oder Isopalmitinsäure ist.

5. Verfahren nach Anspruch 1, **dadurch gekennzeichnet, dass** die C₈- bis C₃₀-Dicarborisäure
• eine Verbindung der Formel (I) 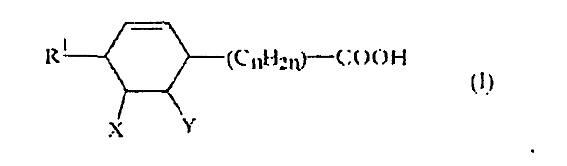 in der R¹ steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest.
• eine Dicarbonsäure der allgemeinen Formel (1), die zusätzlich noch 1 bis 3 Methyl- oder Ethylsubstituenten am Cyclohexenring trägt oder
• eine Dicarbonsäure, die aus den Carbonsäuren gemäß Formel (I) formal durch Anlagerung eines Moleküls Wasser an die Doppelbindung im Cyclohexenring entsteht,
mit der Maßgabe, dass die Substituenten derart gewählt sind, dass die Dicarbonsäure maximal 30 C-Atome enthält, ist.

6. Verfahren nach Anspruch 5, **dadurch gekennzeichnet, dass** die Dicarbonsäure eine Verbindung der Formel (I) ist, bei der R¹ steht für eine Hexylgruppe, n steht für 7. einer der Substituenten X oder Y steht für eine Carboxygruppe und der andere der Substituenten X oder Y steht für Wasserstoff.

7. Mittel zur Farbveränderung keratinischer Fasern, das unmittelbar vor der Anwendung durch Mischen einer ersten alkalisch eingestellten Komponente (A) mit einer zweiten sauer eingestellten Komponente (B), enthaltend mindestens ein Oxidationsmittel, erhalten wird, **dadurch gekennzeichnet, dass** die zweite Komponente (B) mindestens eine verzweigte C₆- bis C₃₀-Fettsäure enthält.

8. Mittel nach Anspruch 7, **dadurch gekennzeichnet, dass** das Oxidationsmittel Wasserstoffperoxid ist.

9. Mittel nach einem der Ansprüche 7 oder 8, **dadurch gekennzeichnet, dass** es weiterhin mindestens ein nichtionisches Tensid enthält.

10. Mittel nach Anspruch 9, **dadurch gekennzeichnet, dass** das nichtionische Tensid ausgewählt ist aus
- Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe. mit einem Methyl- oder C₂ - C₆ - Alkylrest endgruppenverschlossene Anlagerungsprodukten von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe.
- C₁₂-C₃₀-Fettsäuremono- und -diestern von Anlagerungsprodukten von 1 bis 30 Mol Ethylenoxid an Glycerin.
- Ethoxylierung- und Propoxylierungsprodukte von Polyhydoxyfettsäuren.
- Anlagerungsprodukten von 5 bis 60 Mol Ethylenoxid an Rizinusöl und gehärtetes Rizinusöl,
- Polyolfettsäureestern und
- Sorbitanfettsäureestern.

11. Mittel rach Anspruch 10. **dadurch gekennzeichnet, dass** das nichtionische Tensid ausgewählt ist aus Anlagerungsprodukten von 2 bis 50 Mol Ethylenoxid und/oder 0 bis 5 Mol Propylenoxid an lineare und verzweigte Fettalkohole mit 8 bis 30 C-Atomen.

12. Mittel nach einem der Ansprüche 7 bis 11, **dadurch gekennzeichnet, dass** die Komponente (A) mindestens ein Farbstoffvorprodukt enthält.

13. Mittel rach einem der Ansprüche 7 bis 12, **dadurch gekennzeichnet, dass** die fertige Anwendungszubereitung mindestens eine Peroxoverbindung enthält.

14. Mittel nach einem der Ansprüche 7 bis 13, **dadurch gekennzeichnet, dass** die Komponente (A) eine gelförmige Zubereitung ist.

15. Mittel nach einem der Ansprüche 7 bis 14, **dadurch gekennzeichnet, dass** die Komponente (B) mindestens ein Verdickungsmittel enthält.

## Claims

1. Method for the selective colour change of keratinic fibres in which
- in a first step, a first alkaline-rendered component (A) is mixed with a second acidic-rendered component (B), comprising at least one oxidizing agent, to give an application preparation (C),
- in a second step, the resulting application preparation (C) is applied selectively to individual areas of the fibres,
- optionally after a contact time in a third step, all of the fibres are subjected to a colour-changing treatment and
- after a contact time the fibres are rinsed,
**characterized in that** the second component (B) comprises at least one branched C₆- to C₃₀-fatty acid and/or at least one branched or unbranched C₈- to C₃₀- dicarboxylic acid.

2. Method according to Claim 1, **characterized in that** the branched C₆- to C₃₀-fatty acid is saturated.

3. Method according to Claim 2, **characterized in that** the branched C₆- to C₃₀-fatty acid is chosen from 2-ethylhexanoic acid, isotridecanoic acid, isopalmitic acid, isostearic acid, 2-hexyldecanoic acid and 2-octyldodecanoic acid.

4. Method according to Claim 3, **characterized in that** the branched C₆- to C₃₀-fatty acid is isostearic acid or isopalmitic acid.

5. Method according to Claim 1, **characterized in that** the C₈- to C₃₀-dicarboxylic acid is
• a compound of the formula (I) 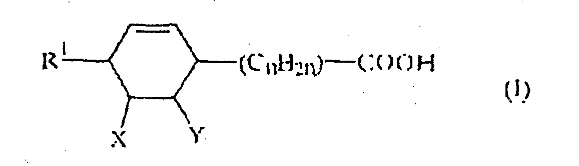 in which R¹ is a linear or branched alkyl or alkenyl group having 4 to 12 carbon atoms, n is a number from 4 to 12, and one of the two groups X and Y is a COOH group and the other is hydrogen or a methyl or ethyl radical,
• a dicarboxylic acid of the general formula (I), which additionally also carries 1 to 3 methyl or ethyl substituents on the cyclohexene ring or
• a dicarboxylic acid which is formed from the carboxylic acids according to formula (I) formally by addition of one molecule of water onto the double bond in the cyclohexene ring,
with the proviso that the substituents are chosen such that the dicarboxylic acid comprises at most 30 carbon atoms.

6. Method according to Claim 5, **characterized in that** the dicarboxylic acid is a compound of the formula (I) in which R¹ is a hexyl group, n is 7, one of the substituents X or Y is a carboxy group and the other of the substituents X or Y is hydrogen.

7. Composition for changing the colour of keratinic fibres which is obtained directly prior to use by mixing a first alkaline-rendered component (A) with a second acidic-rendered component (B) comprising at least one oxidizing agent, **characterized in that** the second component (B) comprises at least one branched C₆- to C₃₀-fatty acid.

8. Composition according to Claim 7, **characterized in that** the oxidizing agent is hydrogen peroxide.

9. Composition according to one of Claims 7 or 8, **characterized in that** it further comprises at least one nonionic surfactant.

10. Composition according to Claim 9, **characterized in that** the nonionic surfactant is chosen from
- addition products of from 2 to 50 mol of ethylene oxide and/or 0 to 5 mol of propylene oxide onto linear and branched fatty alcohols having 8 to 30 carbon atoms, onto fatty acids having 8 to 30 carbon atoms and onto alkyl phenols having 8 to 15 carbon atoms in the alkyl group,
- addition products, terminally capped with a methyl or C₂-C₆-alkyl radical, of from 2 to 50 mol of ethylene oxide and/or 0 to 5 mol of propylene oxide onto linear and branched fatty alcohols having 8 to 30 carbon atoms, onto fatty acids having 8 to 30 carbon atoms and onto alkyl phenols having 8 to 15 carbon atoms in the alkyl group,
- C₁₂-C₃₀-fatty acid mono- and diesters of addition products of from 1 to 30 mol of ethylene oxide onto glycerol,
- ethoxylation and propoxylation products of polyhydroxy fatty acids,
- addition products of from 5 to 60 mol of ethylene oxide onto castor oil and hydrogenated castor oil,
- polyol fatty acid esters and
- sorbitan fatty acid esters.

11. Composition according to Claim 10, **characterized in that** the nonionic surfactant is chosen from addition products of from 2 to 50 mol of ethylene oxide and/or 0 to 5 mol of propylene oxide onto linear and branched fatty alcohols having 8 to 30 carbon atoms.

12. Composition according to one of Claims 7 to 11, **characterized in that** component (A) comprises at least one dye precursor.

13. Composition according to one of Claims 7 to 12, **characterized in that** the finished application preparation comprises at least one peroxo compound.

14. Composition according to one of Claims 7 to 13, **characterized in that** component (A) is a gel-like preparation.

15. Composition according to one of Claims 7 to 14, **characterized in that** component (B) comprises at least one thickener.

## Revendications

1. Procédé de coloration sélective de fibres kératiniques, dans lequel
- dans une première étape, on mélange un premier composant (A) alcalin avec un second composant (B) acide qui contient au moins un agent oxydant, de façon à obtenir une préparation (C) prête à l'emploi,
- dans une deuxième étape, on applique sélectivement la préparation (C) prête à l'emploi que l'on a obtenue sur des zones individuelles de fibres,
- le cas échéant après un temps de pose, dans une troisième étape, on soumet la totalité des fibres à une coloration, et
- après un temps de pose, on rince les fibres,
**caractérisé en ce que** le deuxième composant (B) contient au moins un acide gras ramifié, en C₆ à C₃₀, et/ou au moins un acide dicarboxylique, ramifié ou non ramifié, en C₈ à C₃₀.

2. Procédé selon la revendication 1, **caractérisé en ce que** l'acide gras ramifié en C₆ à C₃₀ est saturé.

3. Procédé selon la revendication 2, **caractérisé en ce que** l'acide gras ramifié en C₆ à C₃₀ est choisi parmi l'acide 2-éthylhexanoïque, l'acide isotridécanoïque, l'acide isopalmitique, l'acide isostéarique, l'acide 2-hexyldécanoïque et l'acide 2-octyldodécanoïque.

4. Procédé selon la revendication 3, **caractérisé en ce que** l'acide gras ramifié en C₆ à C₃₀ est l'acide isostéarique ou l'acide isopalmitique.

5. Procédé selon la revendication 1, **caractérisé en ce que** l'acide dicarboxylique en C₈ à C₃₀ est
• un composé de formule (I) 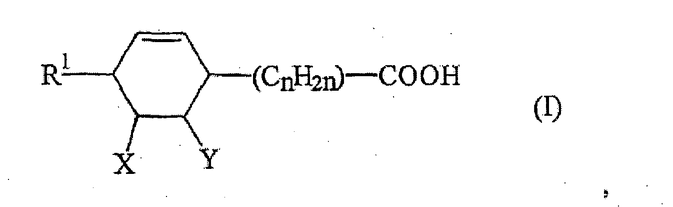 dans laquelle R¹ représente un groupe alkyle ou alcényle linéaire ou ramifié comprenant 4 à 12 atomes de carbone, n représente un nombre de 4 à 12, l'un des deux groupes X et Y représente un groupé COOH et l'autre, un atome d'hydrogène ou un groupe méthyle ou éthyle,
• un acide dicarboxylique de formule (I) dont le noyau cyclohexénique porte en outre 1 à 3 substituants méthyle ou éthyle, ou
• un acide dicarboxylique issu de l'acide carboxylique de formule (I) par addition d'une molécule d'eau sur la double liaison du noyau cyclohexénique,
sous réserve que les substituants soient choisis de façon à ce que l'acide dicarboxylique contienne au maximum 30 atomes de C.

6. Procédé selon la revendication 5, **caractérisé en ce que** l'acide dicarboxylique est un composé de formule (I), dans laquelle R¹ représente un groupe hexyle, n représente le nombre 7, l'un des substituants X ou Y représente un groupe carboxyle et l'autre des substituants X ou Y, un atome d'hydrogène.

7. Agent de coloration de fibres kératiniques, obtenu immédiatement avant application par mélange d'un premier composant (A) alcalin avec un second composant (B) acide qui contient au moins un agent oxydant, **caractérisé en ce que** le second composant (B) contient au moins un acide gras ramifié en C₆ à C₃₀.

8. Agent selon la revendication 7, **caractérisé en ce que** l'agent oxydant est le peroxyde d'hydrogène.

9. Agent selon l'une des revendications 7 ou 8, **caractérisé en ce qu'**il contient en outre au moins un tensioactif non ionique.

10. Agent selon la revendication 9, **caractérisé en ce que** le tensioactif non ionique est choisi parmi
- les produits d'addition de 2 à 50 moles d'oxyde d'éthylène et/ou de 0 à 5 moles d'oxyde de propylène sur des alcools gras linéaires ou ramifiés comprenant 8 à 30 atomes de C, sur des acides gras comprenant 8 à 30 atomes de C et sur des alkylphénols dont le groupe alkyle comprend 8 à 15 atomes de C,
- les produits d'addition de 2 à 50 moles d'oxyde d'éthylène et/ou de 0 à 5 moles d'oxyde de propylène sur des alcools gras linéaires ou ramifiés comprenant 8 à 30 atomes de C, sur des acides gras comprenant 8 à 30 atomes de C et sur des alkylphénols dont le groupe alkyle comprend 8 à 15 atomes de C, dont la chaîne est fermée par un groupe terminal méthyle ou alkyle en C₂ à C₈,
- les mono- et diesters d'acides gras en C₁₂ à C₃₀ de produits d'addition de 1 à 30 moles d'oxyde d'éthylène sur le glycérol,
- les produits d'éthoxylation et de propoxylation d'acides gras polyhydroxylés,
- les produits d'addition de 5 à 60 moles d'oxyde d'éthylène sur l'huile de ricin et l'huile de ricin durcie,
- les esters d'acides gras et de polyols, et
- les esters d'acides gras et de sorbitan.

11. Agent selon la revendication 10, **caractérisé en ce que** le tensioactif non ionique est choisi parmi les produits d'addition de 2 à 50 moles d'oxyde d'éthylène et/ou de 0 à 5 moles d'oxyde de propylène sur des alcools gras linéaires ou ramifiés comprenant 8 à 30 atomes de C.

12. Agent selon l'une quelconque des revendications 7 à 11, **caractérisé en ce que** le composant (A) contient au moins un précurseur de colorant.

13. Agent selon l'une quelconque des revendications 7 à 12, **caractérisé en ce que** la préparation prête à l'emploi contient au moins un composé de type peroxo.

14. Agent selon l'une quelconque des revendications 7 à 13, **caractérisé en ce que** le composant (A) est une préparation sous forme de gel.

15. Agent selon l'une quelconque des revendications 7 à 14, **caractérisé en ce que** le composant (B) contient au moins un agent épaississant.
